# EUROPEAN PATENT APPLICATION

(11) **EP 3 054 390 A1**
(43) Date of publication of application: **10.08.2016**
(21) Application number: 16154102.4
(22) Date of filing: 03.02.2016
(51) Int. Cl.: G06F 19/00

(54) **RADIOGRAPHY IMAGING PARAMETER SELECTION BASED ON EXTANT PATIENT INFORMATION**

(30) Priority: 03.02.2015 US 201514613193
(71) Applicant: Varian Medical Systems International AG, 6330 Cham (CH)
(72) Inventor: PAYSAN, Pascal, 4057 Basel (CH); GRAF, Andres, 4104 Basel (CH)
(74) Representative: Foster, Mark Charles

(57) **Abstract**

A system and method for automatic radiography imaging parameter generation by use of extant patient information is disclosed. The patient information is categorized, and one or more radiography imaging parameters of a radiographic device are selected and processed according to a knowledge-based model applied to a selection of at least one patient information category. A value for the selected imaging parameter(s) is output. Values for several imaging parameters may be automatically generated according to the processing and optimization. Alternatively, a value, or a range of values, may be suggested for presentation to a clinician. The processing and optimization of the radiography imaging parameters may be based on a subset of the patient information.

## Description

### TECHNICAL FIELD

Embodiments of the present disclosure relate generally to imaging devices, and more particularly, to radiographic imaging devices.

### BACKGROUND

Radiation therapy (RT) is a popular and efficient method for cancer treatment, where ionizing radiation is used in an attempt to kill malignant tumor cells or to slow down their growth. RT is often combined with surgery, chemotherapy, or hormone therapy, but may also be used as a primary therapy mode. Radiation therapy may be administered as internal RT or brachytherapy or, more commonly, external beam RT.

Internal RT treatment typically includes placing one or more radioactive sources near a designated treatment area, either permanently or temporarily. Conversely, external beam RT typically involves directing radiation beams produced by sources located externally with respect to the patient or radiation subject to the afflicted treatment area. The beam can consist of photons, electrons, protons or other heavy ions; photons being (at present) the most commonly used particle type. Malignant cells are damaged by the ionizing radiation used during the RT. However, the damage from the radiation is not limited to malignant cells and thus, the dosage of radiation to healthy tissues outside the treatment volume is ideally minimized to avoid those tissues being similarly damaged.

The purpose of traditional RT treatment planning methodologies is to devise a treatment regimen that produces as uniform a dose distribution as possible to the target volumes whilst minimizing the dosage outside this volume. Often, an imaging device such as an X-ray device, Computer Tomography (CT), cone-beam computed tomography (CBCT), or Magnetic Resonance Imaging (MRI) device is used to generate one or more initial scans or images of the area of interest (e.g., pre-treatment planning images). Typically, once an image has been acquired, critical structures (e.g., regions or organs) disposed in the target area are specifically identified so that treatment may be optimally directed.

Typically, immediately prior to a treatment session - but following a pre-treatment planning imaging session - a patient is imaged to confirm patient positioning and provide verification of the location of regions-of-interest for treatment. It is crucial to successful radiation therapy that the discrepancies between dose distributions calculated at the treatment planning stage and those delivered to the patient are minimized. In particular, CBCT integrated with a radiotherapy device can be used for patient positioning and verification in IGRT, especially immediately prior to performing radiation therapy session.

Conventionally, a practitioner must select appropriate source parameters (e.g., source voltage and current, scan trajectory) and image capture parameters (e.g., frame rate, dynamic gain) based on the practitioner's experience and knowledge of the particular patient history. While leveraging human expertise, such an approach is also prone to human error, inconsistency between different practitioners, and a failure to utilize all available information regarding the patient.

### SUMMARY

In one aspect present invention provides a computer implemented method of generating a radiography imaging parameter as defined in the claims.

In another aspect present invention provides a non-transitory computer-readable storage medium embodying instructions as defined in the claims.

In a further aspect present invention provides a radiation therapy device as defined in the claims.

Imager source settings (e.g., radiographic device settings) and image capture settings are ideally optimized for the patient positioning procedure, in order to both acquire a high quality image and at the same time minimize the radiation dose to the patient. Accordingly, embodiments of the present disclosure utilize extant patient-specific information - for example, patient imaging derived from a pre-treatment planning CT scan - in order to automatically generate one or more imaging parameters to be used for imaging the patient just prior to a radiation treatment, e.g., with a kV/MV x-ray imaging system.

This Summary is provided to introduce a selection of concepts in a simplified form that is further described below in the Detailed Description. This Summary is not intended to identify key features or essential features of the claimed subject matter, nor is it intended to be used to limit the scope of the claimed subject matter.

According to embodiments of the present disclosure, a system and method are provided for automatic radiography imaging parameter generation by use of extant patient information. The patient information is categorized, and one or more radiography imaging parameters of a radiographic device are selected and processed according to a knowledge-based model applied to a selection of at least one patient information category. A value for the selected imaging parameter(s) is output. Values for several imaging parameters may be automatically generated according to the processing and optimization. Alternatively, a value, or a range of values, may be suggested for presentation to a clinician. The processing and optimization of the radiography imaging parameters may be based on a subset of the patient information.

More specifically, an aspect of the present disclosure includes a method of generating one or more parameters for a radiography image of a patient. The method includes a step of receiving patient information pertaining to a radiography image for a patient. At least one imaging category is determined, based upon the received patient information. From a number of knowledge-based models, one or more knowledge-based models are selected, the selection made according to the imaging category. From a number of radiography imaging parameters, one or more radiography imaging parameters are selected, the selection made according to the imaging category. The selected radiography imaging parameters are processed according to one or more knowledge-based models for optimization of the selected radiography imaging parameters, and a value of the selected imaging parameter(s) is generated as an output.

The foregoing is a summary and thus contains, by necessity, simplifications, generalizations and omissions of detail; consequently, those skilled in the art will appreciate that the summary is illustrative only and is not intended to be in any way limiting. Other aspects, inventive features, and advantages of the present invention, as defined solely by the claims, will become apparent in the non-limiting detailed description set forth below.

### BRIEF DESCRIPTION OF DRAWINGS

The accompanying drawings, which are incorporated in and form a part of this specification, illustrate embodiments of the invention and, together with the description, serve to explain the principles of the invention:
Figure 1 depicts an exemplary radiation system, in accordance with an embodiment of the present disclosure.
Figure 2 depicts a functional block diagram illustrating an exemplary configuration of a radiography imaging parameter generation system in accordance with an embodiment of the present disclosure.
Figure 3 depicts a flowchart of a method of generating an imaging parameter for a radiography imaging device, in accordance with an embodiment of the present disclosure.
Figure 4 depicts a flowchart of a method of generating an imaging parameter for a radiography imaging device, including optional consideration of a subset of patient data and/or manual selection of a knowledge-based model, in accordance with an embodiment of the present disclosure.
Figure 5 depicts an exemplary computing environment, in accordance with embodiments of the present disclosure.

### DETAILED DESCRIPTION

Reference will now be made in detail to several embodiments. While the subject matter will be described in conjunction with the alternative embodiments, it will be understood that they are not intended to limit the claimed subject matter to these embodiments. On the contrary, the claimed subject matter is intended to cover alternative, modifications, and equivalents, which may be included within the spirit and scope of the claimed subject matter as defined by the appended claims.

Furthermore, in the following detailed description, numerous specific details are set forth in order to provide a thorough understanding of the claimed subject matter. However, it will be recognized by one skilled in the art that embodiments may be practiced without these specific details or with equivalents thereof. In other instances, well-known methods, procedures, and components, have not been described in detail as not to unnecessarily obscure aspects and features of the subject matter.

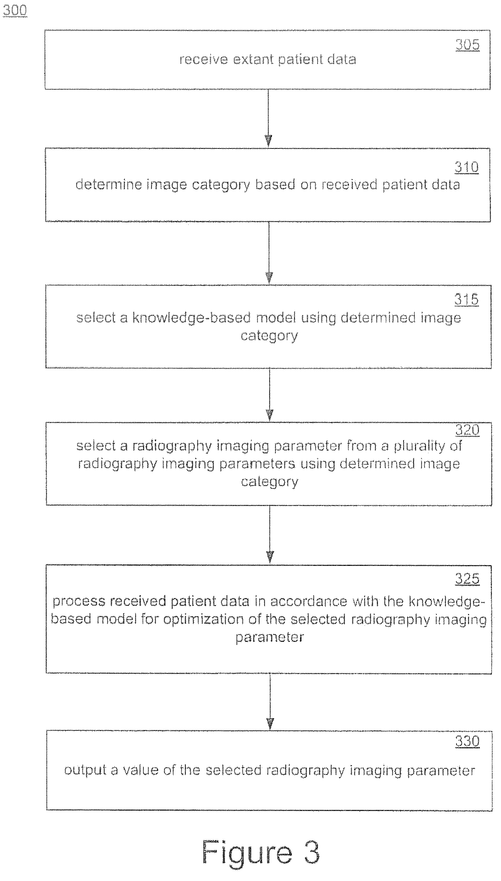

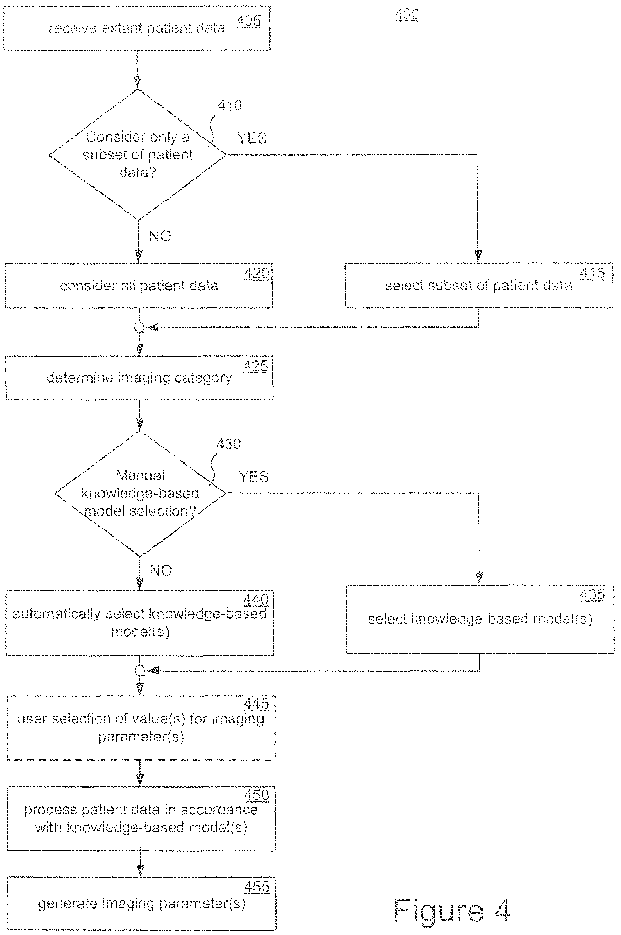

Portions of the detailed description that follows are presented and discussed in terms of a method. Although steps and sequencing thereof are disclosed in figures herein (e.g., Figures 3 and 4) describing the operations of this method, such steps and sequencing are exemplary. Embodiments are well suited to performing various other steps or variations of the steps recited in the flowchart of the figure herein, and in a sequence other than that depicted and described herein.

Embodiments described herein may be discussed in the general context of computer-executable instructions residing on some form of computer-usable medium, such as program modules, executed by one or more computers or other computing devices. Generally, program modules include routines, programs, objects, components, data structures, etc., that perform particular tasks or implement particular abstract data types. The functionality of the program modules may be combined or distributed as desired in various embodiments.

By way of example, and not limitation, computer-usable media may comprise computer storage media and communication media. Computer storage media includes volatile and nonvolatile, removable and non-removable media implemented in any method or technology for storage of information such as computer-readable instructions, data structures, program modules or other data. Computer storage media includes, but is not limited to, random access memory (RAM), read only memory (ROM), electrically erasable programmable ROM

(EEPROM), flash memory or other memory technology, compact disk ROM (CD-ROM), digital versatile disks (DVDs) or other optical storage, magnetic cassettes, magnetic tape, magnetic disk storage or other magnetic storage devices, or any other medium that can be used to store the desired information.

Communication media can embody computer-readable instructions, data structures, program modules or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, radio frequency (RF), infrared and other wireless media. Combinations of any of the above should also be included within the scope of computer-readable media.

As used herein, the term "image" or "medical image" may be used interchangeably and refers to an image created by an imaging system, which includes but is not limited to x-ray radiography, X-ray computed tomography (CT) imaging, cone-beam computed tomography (CBCT) imaging, magnetic resonance imaging (MRI), positron emission tomography (PET) imaging, single photon emission computed tomography (SPECT) imaging, and ultrasound (US) imaging. A medical image can be either a 2D image or a 3D image.

In the following embodiments, a technique to automatically generate radiography imaging parameters for a patient is described. Embodiments include a method for generating one or more radiography imaging parameters, based on extant patient information and in accordance with a knowledge-based model.

### EXEMPLARY RADIATION TREATMENT AND IMAGING MACHINE

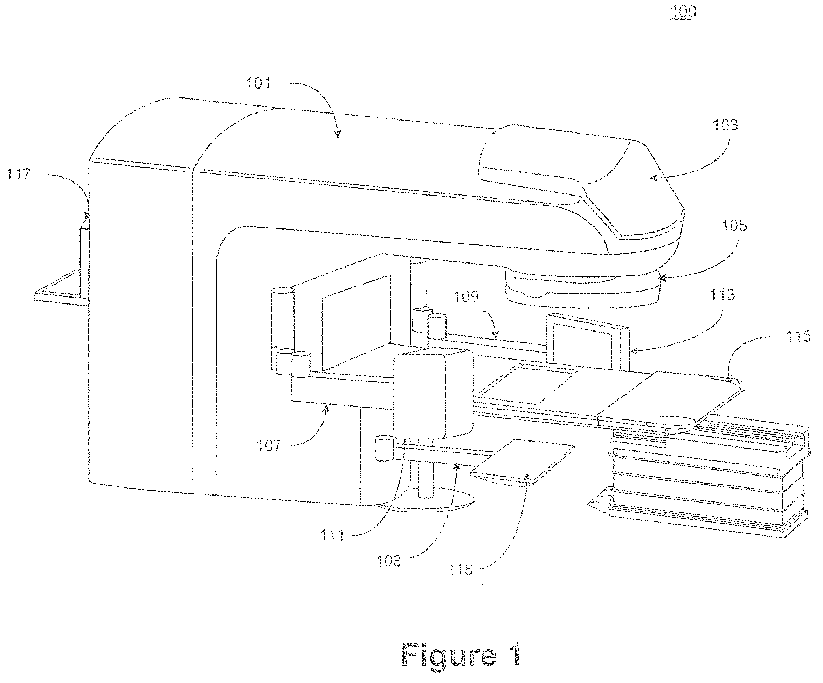

With reference now to Figure 1, an illustration of an exemplary radiation therapy and imaging device 100 is depicted, in accordance with one embodiment of the present disclosure. In one configuration, radiation therapy and imaging device 100 includes a support structure (e.g., gantry 101), and a radiation source (e.g., a medical or clinical linear accelerator) including a treatment head 103. In one embodiment, the treatment head 103 is adapted to receive a primary electron beam (e.g., from an electron gun), which upon incidence with a target material (commonly consisting of a high-Z metal) will produce what are referred to as "bremsstrahlung photons," and thereby generate a photon beam, ultimately emanating from a treatment source 105.

In further embodiments, a plurality of robotic arms (e.g., robotic arms 107, 108 and 109) may be attached to the gantry and coupled to a second (diagnostic) radiation source 111, and an area detector panel 113 (typically comprising a grid of single point detectors). According to some embodiments, an additional arm may be attached to a portal area detector panel 118 for receiving radiation from the treatment source 105. In still further embodiments, the device 100 may also include a patient couch 115. The couch 115 in an embodiment is a robotic couch, able to move in order to change a position of a patient on the couch. The radiation therapy and imaging device 100 may further include a communicatively coupled computing device 117 for processing images and/or controlling and manipulating the device 100. According to some embodiments, radiation emanating from the treatment source 105 and received in the portal area detector panel 118 may operate in the range of MV radiation (mega-voltage radiation), whereas radiation distributed from the second radiation source 111 and received in the area detector panel 113 may operate in the range of kV radiation (kilo-voltage).

In one embodiment, the end of gantry 101 positioned above patient couch 115 is attached to a treatment source 105 used to generate radiation (e.g., for therapeutic or imaging purposes). A patient is positioned (typically supine) on patient couch 115. A target volume (generally disposed within or about the patient subject) is irradiated by transmitting a radiation beam (e.g., X-ray photon beam) through the target volume and receiving the beam in the area detector panel 113 from source 111 or detector panel 118 from source 105.

According to one embodiment, an image of the target volume can be acquired using either the diagnostic source 111 or therapeutic radiation source 105 in conjunction with either the area detector 113 or area detector 118, respectively. In alternate embodiments, x-ray detectors and radiation sources may be used for diagnostic and other applications that are not related to the radiotherapy field. In an embodiment, data corresponding to radiation energy received for each radiographic image are stored by computing device 117. In a further embodiment a memory comprised by computing device 117 is substantially radiation-hardened, using substrates and/or logical methods known to those skilled in the art.

In other embodiments, the system may not include a radiation therapy device. Instead, the system may consist of a radiation source and an imager. For example, in other embodiments, the system may include an x-ray source and a flat panel detector. In further embodiments, the source and imager may or may not be mounted on a rotational gantry (e.g., rotational gantry for CBCT). While embodiments are described herein to comprise the generation and usage of X-ray (photon) beams, it is to be understood that alternate embodiments are well suited for the generation and use of other particles and corresponding particle beams. These alternate embodiments may include, but are not limited to, electrons and electron beams; protons and proton beams; and ions and ion beams.

According to embodiments of the present disclosure, radiography imaging parameters are generated for imaging that is performed for registration (that is, positioning) of the patient, just prior to a treatment session. Imaging parameters may be suggested, or preselected, according to a determined optimization. Patient information may be categorized in order to direct the selection of radiography imaging parameters for optimization. For example, patient information about the weight of the patient, materials present (such as metal), what is to be optimized-e.g., optimization of dose for a child, where induced cancer is a danger, versus image contrast for soft tissue (which requires greater radiation dose) - may all be factored into imaging parameter generation. The registration of the patient may be established according to optimization of regions of interest - e.g., if a target area is of a bony structure, imaging performed may be optimized for locating bony structures only (lower radiation dose required, since higher contrast for bony structures). Or, if the target area is of soft tissue, greater radiation dose may be necessary.

According to embodiments of the present disclosure, certain radiography imaging parameters of the device 100 may be automatically calibrated (or alternatively, suggested) to produce or emphasize certain qualities of generated images. These parameters may include, for example, modifying the intensity (e.g., source voltage and/or source current) or geometry (e.g., angle or trajectory) of the photon beam, and/or position of area detector 113 or area detector 118. A geometric image acquisition parameter may include a source and/or detector position, orientation, and/or trajectory, a scan trajectory, a fan type selection (e.g., half- or full-fan), and a position of couch 115. Additionally, a beam pulse length of the radiation source may be controlled, as well as a focus mode. In one aspect, an imaging and/or matching mode can be automatically selected, or alternatively, suggested. Imaging parameters may encompass, for example: determination of capturing a static volume or a sequence of multiple volumes (e.g., 4D-CBCT); a Digital tomosynthesis (DTS) setting; a CBCT setting; determination of a gating setting (e.g., synchronization of imaging with a phase of patient anatomy movement); and an image reconstruction parameter (e.g., image slice thickness), as a non-exhaustive list.

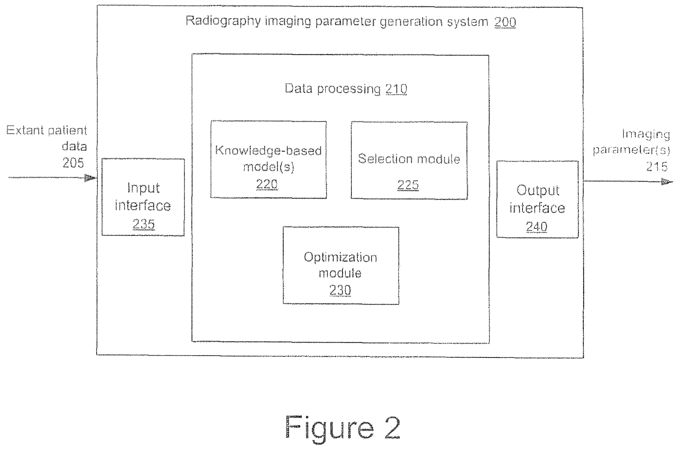

Image acquisition parameters may include, for example: detector frame rate; filter and bowtie selection; settings for collimator blades, and/or a multileaf collimator (MLC); detector settings such as gain, dark field, and binning. Other image processing parameters known to those of skill in the art are consistent with the spirit and scope of the present disclosure. Further, according to embodiments of the present disclosure, control of one or more functions of an external device (or devices) in communication with the imaging device. Exemplary external device functionality includes: gating; a surrogate; a motion management device; and an external positioning device. The manner of automatic generation for these and other radiography imaging parameters is described further herein, especially in the description of Figures 2-5.

In one aspect, embodiments of the present disclosure employ all of the patient data from pre-treatment steps in order to optimize radiography imaging parameters for a patient positioning image capture. The radiography imaging parameters may include both source parameters (e.g., source voltage) and image capture parameters (e.g., frame rate). To "optimize" may entail optimization of one or more imaging parameters, depending on the nature of the patient case and what the desired outcome may be.

Referring now to Figure 2, a functional block diagram depicts an exemplary configuration of a radiography imaging parameter generation system 200 in accordance with an embodiment of the present disclosure. The radiography imaging parameter generation system 200 includes a data processing component 210 that implements one or more knowledge-based model(s) 220, a selection module 225, and an optimization module 230. The radiography imaging parameter generation system 200 may optionally further include an input interface 235 and output interface 240. The system 200 in whole or in part may be implemented as a software program, hardware logic, or a combination thereof.

Knowledge-based models 220 are able to include expert systems and/or physical models. An expert system has knowledge of the particular patient case, and depending on the case, the system proposes pre-set parameters. For example, an expert system can be implemented as a database of different cases, and for each case, unique parameters are specified. Each case in the database can included image pixel data, the image pixel data including anatomical information such as contours of anatomical structures, bone diameters, an amount of water present, fat tissue present, size of lung tissue, a tumor contour, etc. Typically an expert system exists at the subsystem level - that is, a metal expert for a particular metal exists (e.g., parameters chosen to optimize imaging of metal), and for a patient having a metal implant the metal expert for the corresponding metal implant material may be used. Likewise, experts exist for varying types of soft-tissue, for bony tissue, etc. Different combinations of expert models can together select the imaging parameters for a present case.

In contrast, a physical model possesses knowledge about the particular materials present in imaging volume (e.g., an amount of bone, an amount of metal, an amount of soft-tissue identified in the image pixel data of the case). Based on the information regarding the materials in the imaging volume, one or more physical models can be applied in order to determine a radiography imaging parameter (e.g., a source voltage).

Employing a combination of expert and physical model protocols is possible. In operation, extant patient data 205 (that is, patient data that exists prior to the positioning imaging session) is provided to the radiography imaging parameter generation system 200, preferably via the input interface 235. The input patient data 205 may contain any combination of parameters that can practically affect the radiography imaging and may be organized as a vector or other data structure. The patient data 205 is able to include various kinds of information, such as: a patient record (e.g., age, disease type); patient measurements (e.g., weight, height, body outline from an optical system measurement); a radiation therapy plan; pre-treatment planning CT data; a body region for treatment (e.g., head, thorax, pelvis); an image purpose and/or application, to name a few. Other patient-specific information known to those of skill in the art is consistent with the spirit and scope of the present disclosure. Based on the patient data 205, one or more imaging categories for the radiography image are determined. The one or more imaging categories can include a type of patient to be imaged (e.g., a pediatric patient, an obese patient), as well as an anatomical region to be imaged (e.g., head, thorax, pelvis). In general, an imaging category is able to be specified such that it is discriminative with regard to the radiography imaging parameters that are optimally generated for the patient. That is, a pediatric patient should have a different imaging protocol than an adult, and likewise an image of the thorax (which is likely moving during imaging) may require a different protocol than an image of the head.

Determination of the imaging category (or categories) enables selection of one or more radiography imaging parameters to be optimized for the image, the selection performed by selection module 225. According to an embodiment of the present disclosure, the selection module 225 can automatically select the one or more radiography imaging parameters. The selection module 225 is further configured to identify and select one or more knowledge-based models, based on the patient data 205 and the determined imaging category, the selected knowledge-based model for determining an imaging protocol suitable for the present patient. The selected one or more radiography imaging parameters are processed according to the selected knowledge-based model (e.g., an expert model), in order to generate a value for the selected radiography imaging parameters, which is output as imaging parameter(s) 215.

The selection of the radiography imaging parameters, and of the knowledge-based model, may be transparent to a user, e.g., a therapy planner or practitioner, such that manual selection is unnecessary. Alternatively, as described herein (e.g., at Figures 3 and 4), manual selection of certain radiography imaging parameters and knowledge-based models for processing is consistent with the spirit and scope of the present disclosure. In some embodiments, the system may comprise a user interface (e.g., input interface 235 and output interface 240) that allows a user to narrow down one or more of the radiography imaging parameters, the patient data considered, and the knowledge-based models used, by user-defined constraints. Alternatively, the system may restrict parameters, so that user may only select values within a certain range (determined by system according to planning knowledge). Alternatively, the system may suggest parameters (e.g., based on planning data showing a head scan, the system may generate radiography imaging parameters corresponding to a head protocol). In an embodiment, the system 200 may be implemented "offline," and determination of the proper imaging parameters is able to be made prior to the patient treatment.

As will be appreciated by those skilled in the art, the present disclosure is not limited to any mechanism or criteria of determining an imagine category and/or suitable knowledge-based model based on patient data. The selection could be based on a similarity between the patient data 205 and an expert system and/or physical model included in knowledge-based models 220. The selection could be based on maximum similarity, e.g., only one imaging category (or knowledge-based model) is selected, the one with the highest similarity according to a chosen similarity metric with the patient data 205. Alternatively, the selection could also be based on certain acceptable similarity level, e.g., the number of selected imaging categories (or knowledge-based models) could differ, where all imaging categories (or knowledge-based models) with sufficiently high similarity are selected. For example, bony matches are often performed for patient positioning imaging in pre-treatment. For a bony match soft tissue contrast is not particularly important, and therefore a knowledge-based model selection that is based on only one expert model (or physical model) can be made, that knowledge-based model including a protocol that reduces the radiation dose substantially in order to obtain only the image of the bone.

The individual knowledge-based models 220 may originate from a clinic having several models to cover different regions, or be developed by a radiation equipment provider, or are shared among several clinics. The models may be derived from published literature data or clinical data as submitted by clinic practitioners.

### RADIOGRAPHY IMAGING PARAMETER GENERATION

Referring now to Figure 3, a flowchart 300 is depicted of one embodiment of a method for generating one or more radiography imaging parameters for a radiography image of a patient, based on extant patient information. Method 300 can be implemented as a system, for example, the system 200 illustrated in Figure 2. Steps 305-330 describe exemplary steps comprising the process depicted in flowchart 300 in accordance with the various embodiments herein described. In one embodiment, the flowchart 300 is implemented as computer-executable instructions stored in a computer-readable medium.

At step 305, patient information (e.g., patient data 205) is received at a system configured to generate radiography imaging parameters to be used for imaging the patient by a radiographic device. The patient data is able to include various kinds of information, such as: a patient record (e.g., age, disease type); patient measurements (e.g., weight, height, body outline from an optical system measurement); a radiation therapy plan; pre-treatment planning CT data; a body region for treatment (e.g., head, thorax, pelvis); an image purpose and/or application, to name a few. Other patient-specific information known to those of skill in the art is consistent with the spirit and scope of the present disclosure.

Once the patient data is received, a determination of one or more imaging categories to which the received patient information belongs is made at step 310. As described herein, an imaging category can include a type of patient to be imaged (e.g., a pediatric patient, an obese patient), as well as an anatomical region to be imaged (e.g., head, thorax, pelvis). In general, an imaging category is able to be specified such that it is discriminative with regard to the radiography imaging parameters that are optimally generated for the patient. That is, a pediatric patient has a different imaging protocol than an adult, and likewise an image of the thorax (which is likely moving during imaging) may require a different protocol than an image of the head.

At step 315 a selection is made of a knowledge-based model (e.g., knowledge-based model 220), the selection based on the imaging category determined at step 310. As described herein, the knowledge-based model can include expert systems and/or physical models. The knowledge-based model(s) may be automatically selected, and can be an expert model for a particular subsystem - e.g., radiography imaging parameters optimized for imaging soft-tissue, or for bony tissue, etc. Alternatively or additionally, the knowledge-based system is based on physical model, where knowledge exists about the materials present in imaging volume (e.g., an amount of bone, an amount of metal, an amount of soft-tissue, etc.). A plurality of expert models and physical models are able to be selected, and combinations of expert and physical models are also consistent with the spirit and scope of the present disclosure. According to embodiments of the present disclosure, the selection of the knowledge-based model can be automatic, or alternatively, a user of the system can perform manual selection (e.g., via input interface 235).

At step 320, a selection is made of one or more radiography imaging parameters, where the radiography imaging parameter(s) are selected using the determined imaging category. There are several factors that affect both the imaging quality and the imaging parameters to be used. These include the structures (like bone, or a high amount of soft tissue, e.g., pelvis region) surrounding the target image volume. Additionally, anticipated motion of the subject (due to, for example, breathing) affects image quality and acquisition, as well as the acquisition geometry. For certain target areas, selection of an imaging parameter is made to extend a scan radius - e.g., for head, an imaging modality with a smaller radius is used (e.g., full-fan mode), whereas for pelvis, a larger field of view is desired (e.g., half-fan mode).

According to an embodiment of the present disclosure, selecting a radiography imaging parameter includes a user interaction (e.g., via input interface 235), enabling user-specified values to be used for generating imaging parameters. For example, a user is able to specify a specific value or range of values for one or more imaging parameters, with other imaging parameters being generated while taking the user-specified parameter value(s) into account.

At step 325, the selected radiography imaging parameters are processed in accordance with one or more knowledge-based models for optimization of the radiography imaging parameter(s) selected at step 320. The knowledge-based model functions to determine a value for the one or more radiography imaging parameters, specifically the radiography imaging parameters from step 320. For example, for an image of thorax, motion of the thorax is assumed, and therefore an expert system will determine radiography imaging parameters that include the use of 4D-CBCT (which considers breathing motion), and/or gating. That is, in order to minimize effect of patient motion, a knowledge-based model will process the patient data in order to generate an imaging protocol which is very short. Conversely, the knowledge-based model can indicate the use of gating, acquiring an image only if patient is in maximum inhale phase, for example. If the image were of a patient head, a different protocol would be selected/suggested by the knowledge-based model.

In the case of the physical model, radiography imaging parameters affected may be an imager frame rate, filter/bowtie selection, a source current/voltage, among others. In some embodiments, an expert system is able to determine certain classes of parameters (e.g., gating, 4D-CBCT, etc.), while a physical model influences frame rate, etc., as above. A combination of the expert system and the physical model may be used to determine a suite of parameter values that are generated.

At step 330, an output is made of a generated value for the selected radiography imaging parameter(s). In an embodiment, one value is generated for each radiography imaging parameter selected in step 315. In an embodiment, a range of values are generated for each radiography imaging parameter, or for a select number of a plurality of radiography imaging parameters. According to embodiments of the present disclosure, the value (or range of values) may be suggested values, or they may be automatically applied to the imaging system (e.g., a radiographic device and imaging system). According to embodiment, the values may be excluded values.

Referring now to Figure 4, a flowchart 400 depicts one embodiment of a method of generating one or more radiography imaging parameters for a radiography image of a patient, including optional selection of a subset of extant patient information for processing by select knowledge-based models. The process 400 includes optional user selection of certain imaging parameter values, with other imaging parameters being generated with respect to the user-specified values. Steps 405-455 describe exemplary steps comprising the process depicted in flowchart 400 in accordance with the various embodiments herein described. In one embodiment, the flowchart 400 is stored in a computer-readable medium.

At step 405 extant patient data is received. The patient data is able to include various kinds of information, such as: a patient record (e.g., age, disease type); patient measurements (e.g., weight, height, body outline from an optical system measurement); a radiation therapy plan; pre-treatment planning CT data; a body region for treatment (e.g., head, thorax, pelvis); an image purpose and/or application, to name a few. Other patient-specific information known to those of skill in the art is consistent with the spirit and scope of the present disclosure.

At step 410 a determination is made of whether only a subset of the patient data received at step 405 will be considered. If NO, the process continues to step 420 and considers all of the patient data received at step 405. If YES, the process continues to step 415, where the subset of patient data to be considered by process 400 is selected. The selection may be made by an operator of a system performing the process 400, via input interface 235 for example. Alternatively, the selection may be predetermined, and the predetermined subset of patient data may be provided. For example, one or more categories of patient data may be identified for subset selection (e.g., patient record and radiation therapy plan) while others are identified to be ignored (e.g., image purpose and patient measurements). In this manner a relative importance of different aspects (e.g., categories) of the patient data can be indicated by a practitioner, or any user of a system implementing process 400.

Once the patient data for consideration is determined, a determination of one or more imaging categories is made at step 425. As described herein, an imaging category can include a type of patient to be imaged (e.g., a pediatric patient, and obese patient), as well as an anatomical region to be imaged (e.g., head, thorax, pelvis). In general, an imaging category is able to be specified such that it is discriminative with regard to the radiography imaging parameters that are optimally generated for the patient.

At step 430 a determination is made of whether a knowledge-based model (or models) is to be selected manually. If NO, the process continues to step 440, where automatic selection of one or more knowledge-based models occurs. If YES, the process continues at step 435, where a selection is made of the particular knowledge-based model (or models) that will be used for processing the patient data from step 415 or 420. For example, the knowledge-based model can be an expert model for a particular subsystem - e.g., radiography imaging parameters optimized for imaging soft-tissue, or for bony tissue, etc. Alternatively or additionally, the knowledge-based system is based on physical model, where knowledge exists about the materials present in imaging volume (e.g., an amount of bone, an amount of metal, an amount of soft-tissue, etc.). A plurality of expert models and physical models are able to be selected, and combinations of expert and physical models are also consistent with the spirit and scope of the present disclosure. In an embodiment, the selection may be made by an operator of a system performing the process 400, via input interface 235 for example. Alternatively, the selection may be predetermined based upon, for example, the knowledge of a practitioner regarding the specific history and disease of a patient for whom a positioning image is being planned.

An optional step 445 includes a user selection of one or more imaging parameters (e.g., via input interface 235), enabling user-specified values to be used. In one respect, user-specified values of given imaging parameters cause the process 400 to generate other imaging parameters (in step 455) with respect to the user-specified imaging parameters. As a non-limiting example, a user specification of a full-fan image capture mode (due to, for example, a scan of the neck) can cause the process 400 to generate other imaging parameters around the full-fan constraint, leading to specified source/detector orientations and scan trajectories.

Following the selection of the particular knowledge-based model(s), at step 450 the patient data is processed in accordance with the selected knowledge-based model(s). At step 455, one or more radiography imaging parameters are generated. Different combinations of expert systems can together select the radiography imaging parameters for the patient. According to embodiments of the present disclosure, the generated imaging parameter(s) from step 455 are suggestions presented to a user (e.g., via output interface 240). The suggested imaging parameter(s) are able to be confirmed by a user, or alternatively, the suggested imaging parameter(s) can be changed from the suggested value to a user-specified value. The suggested imaging parameter(s) can be presented as a specified value (e.g., source oriented at 30 degrees), or as a range of values (e.g., source current between 60-80 mA).

Embodiments of the present disclosure may be appreciated by the use of exemplary imaging cases. In one example, for a young person (e.g., a pediatric case) it is appropriate to optimize radiography imaging parameters such that a reduced radiation dose is delivered. Imaging parameters that are appropriate to select in this case are a setting of a lower source voltage, as well as a lower source current. Additionally, a shorter beam pulse length may automatically be selected (or suggested, in some embodiments). If higher soft-tissue contrast is indicated (e.g., by a practitioner) the optimization of these radiography imaging parameters could include an increase in one or more of these parameter values.

In one example, for an obese patient image parameter selection and optimization can be made to avoid image truncation. Truncation occurs when less than 100% of a target imaging volume is successfully imaged. From patient data 205 including a planning CT of the patient, the weight of the patient, body outline, etc. are known. The patient data 205 includes the body weight stated in patient record, as well as information about the body part that is going to be imaged. These can be used to estimate the necessary dose to image the body part. It is possible to avoid truncation by selecting and optimizing parameters that include moving the couch (e.g., couch 115), for example. Alternatively or additionally, the detector (e.g., detector 113 and/or detector 118) may be moved in order to change the imaging volume. Reduced detector motion can also lead to acquiring an image more quickly, as the imaged volume may be reduced. Conversely, moving the detector allows imaging of a larger volume, at the cost of taking more time. Additionally, for a couch 115 that is robotic, the patient can be moved around to be placed in an optimal position. Therefore the volume imaged may be changed via imaging parameters including detector movement, couch movement, and a combination of both detector and couch movement.

Another exemplary case involves a treatment plan (e.g., from patient data 205) that indicates fractionated treatments. Often, a treatment is fractionated into a number of fractions. For example, a full treatment regimen may be broken down into 30 fractions so that, for a total dose of 30 units, there is one (1) dose unit per fraction. Each fraction may be performed on a given day - so, for 30 fractions, the treatment is completed in 30 days. For a fractionated treatment plan, the required accuracy of dosage delivery is dependent to some degree on the number of fractions. For a large number of fractions (highly fractionated), since there will be more occurrences of radiation delivery, each individual radiation dose is reduced and the concomitant need for accuracy in delivery is lessened. Conversely, if few fractions are used, this is greater dose per fraction and a concomitant greater accuracy in dose delivery to the target area is needed. This means that the pre-treatment imaging is affected, in the sense that more time and/or a greater dose may be used for placement image when lower fractionation is used, and less time and/or a lower dose may be used for placement image when greater fractionation is used. According to an embodiment, an input parameter to the system 200 includes what the tolerance for the imaging dose may be.

A function of the system and methods of the present disclosure is to provide prevention of an image fault, for example, an image overexposure due to operator error. If an operator applies too much dosage (e.g., dosage applied for a maximum volume when a maximum volume is not present), the image will be overexposed. Knowledge of the volume being imaged, from patient data 205, enables guidelines on a particular dosage range for that image. Embodiments according to the present disclosure are able to place a limit on applied dose in order to prevent overexposure/underexposure.

### EXEMPLARY COMPUTING DEVICE

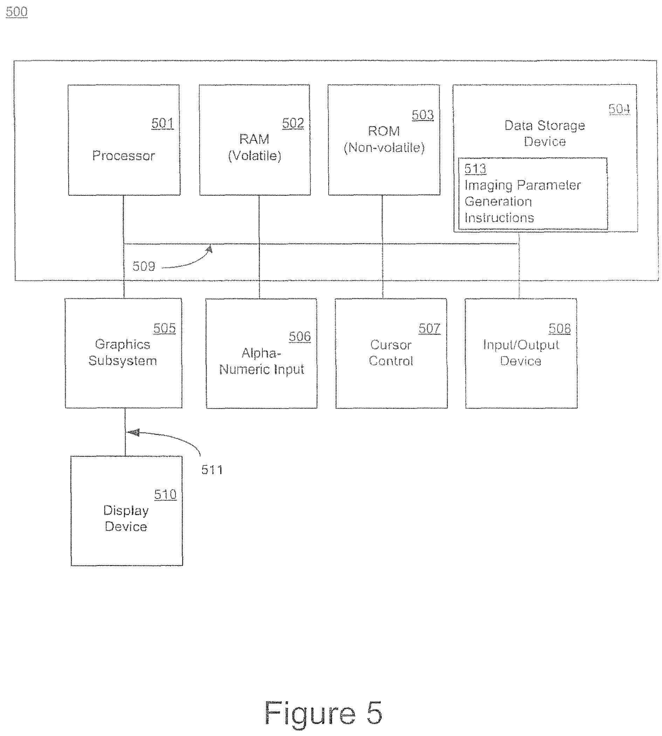

As presented in Figure 5, an exemplary system upon which embodiments of the present disclosure may be implemented includes a general purpose computing system environment, such as computing system 500. In its most basic configuration, computing system 500 typically includes at least one processing unit 501 and memory, and an address/data bus 509 (or other interface) for communicating information. Depending on the exact configuration and type of computing system environment, memory may be volatile (such as RAM 502), nonvolatile (such as ROM 503, flash memory, etc.) or some combination of the two. In some embodiments the memory is substantially radiation-hardened, using substrates and/or logical methods known to those skilled in the art.

Computer system 500 may also comprise an optional graphics subsystem 505 for presenting information to the computer user, e.g., by displaying information on an attached display device 510, connected by a video cable 511. According to embodiments of the present disclosure, the graphics subsystem 505 may be coupled directly to the display device 510 through the video cable 511. A graphical user interface of an application for controlling a medical linear accelerator executing in the computer system 500 may be generated in the graphics subsystem 505, for example, and displayed to the user in the display device 510. In alternate embodiments, display device 510 may be integrated into the computing system (e.g., a laptop or netbook display panel) and will not require a video cable 511.

Additionally, computing system 500 may also have additional features/functionality. For example, computing system 500 may also include additional storage (removable and/or non-removable) including, but not limited to, magnetic or optical disks or tape. Such additional storage is illustrated in Figure 5 by data storage device 507. Computer storage media includes volatile and nonvolatile, removable and non-removable media implemented in any method or technology for storage of information such as computer readable instructions, data structures, program modules or other data. RAM 502, ROM 503, and data storage device 507 are all examples of computer storage media.

In an embodiment, computer system 500 comprises instructions for performing processes according to aspects of the present disclosure, where the instructions may be stored on RAM 502, ROM 503, and/or data storage 504. For example, the computer system 500 may comprise radiography imaging parameter generation instructions 513, where radiography imaging parameter generation instructions 513 contain instructions causing computer system 500 to perform a process of generating a value for one or more radiographic imaging parameters automatically from extant patient information, according to embodiments of the present disclosure (e.g., processes 300, 400).

Computer system 500 also comprises an optional alphanumeric input device 506, an optional cursor control or directing device 507, and one or more signal communication interfaces (input/output devices, e.g., a network interface card) 509. Optional alphanumeric input device 506 can communicate information and command selections to central processor 501. Optional cursor control or directing device 507 is coupled to bus 509 for communicating user input information and command selections to central processor 501. Signal communication interface (input/output device) 509, also coupled to bus 509, can be a serial port. Communication interface 509 may also include wireless communication mechanisms. Using communication interface 509, computer system 500 can be communicatively coupled to other computer systems over a communication network such as the Internet or an intranet (e.g., a local area network), or can receive data (e.g., a digital television signal).

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A computer implemented method of generating a radiography imaging parameter, the method comprising:
receiving patient information pertaining to a radiography image for a patient;
determining an imaging category based upon the received patient information;
selecting at least one knowledge-based model using the determined imaging category;
selecting at least one radiography imaging parameter from a plurality of radiography imaging parameters of a radiographic device using the determined imaging category;
processing the received patient information in accordance with the knowledge-based model for optimization of the at least one selected radiography parameter; and
outputting a value of the at least one selected radiography imaging parameter.

2. The computer implemented method according to Claim 1, wherein the patient information is based on at least one of: a therapy planning imaging session data; a patient health record; a radiation therapy plan; a pre-treatment planning computed tomography image parameter; a body region of the patient.

3. The computer implemented method according to Claim 1 or 2, wherein the knowledge-based model includes at least one of an expert system and a physical model.

4. The computer implemented method according to Claim 1, 2 or 3, wherein the plurality of radiography imaging parameters comprises: a radiographic device imaging mode; an image capture parameter; a geometric image acquisition parameter; a body region of the patient; at least one of a radiographic device source voltage and a radiographic device source current; a control operation of a device external to a radiographic device.

5. The computer implemented method according to Claim 1, 2, 3 or 4, wherein the outputting a value comprises a suggested value for the selected radiography imaging parameter.

6. A non-transitory computer-readable storage medium embodying instructions that, when executed by a processing device, cause the processing device to perform a method of:
receiving patient information pertaining to a radiography image for a patient;
determining an imaging category based upon the received patient information;
selecting a knowledge-based model using the determined imaging category;
selecting at least one radiography imaging parameter from a plurality of radiography imaging parameters of a radiographic device using the determined imaging category;
processing the received patient information in accordance with the knowledge-based model for optimization of the at least one selected radiography parameter; and
outputting a value of the at least one selected radiography imaging parameter

7. The non-transitory computer-readable storage medium according to Claim 6, wherein the patient information is based on at least one of: a therapy planning imaging session data; a patient health record; a radiation therapy plan; a pre-treatment planning computed tomography image parameter; a body region of the patient.

8. The non-transitory computer-readable storage medium according to Claim 6 or 7, wherein the plurality of radiography imaging parameters comprises: a radiographic device imaging mode; an image capture parameter; a geometric orientation of image capture; a body region of the patient; at least one of a radiographic device source voltage and a radiographic device source current; a control operation of a device external to a radiographic device.

9. The non-transitory computer-readable storage medium according to Claim 6, 7 or 8, wherein the outputting a value comprises restricting the at least one selected radiography imaging parameter to a range of values.

10. The non-transitory computer-readable storage medium according to Claim 6, 7, 8 or 9, wherein the knowledge-based model is comprised by a plurality of knowledge-based models, and the knowledge-based model of the plurality of knowledge-based models is automatically selected for the processing the received patient information.

11. A radiation therapy device comprising:
a plurality of radiation sources;
a plurality of imagers for receiving radiation from the plurality of radiation sources and configured to generate data representing a target image comprising a plurality of anatomical structures; and
a computing device configured to access patient information pertaining to a radiography image for a patient, the computing device configured to determine an imaging category based upon the patient information and to select a knowledge-based model based upon the determined imaging category, and to select at least one radiography imaging parameter from a plurality of radiography imaging parameters of the radiation therapy device, the radiography imaging parameter selection based upon the imaging category, wherein the computing device is configured to process the received patient information in accordance with the knowledge-based model for optimization and to generate a value of the at least one selected radiography imaging parameter.

12. The radiation therapy device according to Claim 11, wherein the patient information is based on at least one of: a therapy planning imaging session data; a patient health record; a radiation therapy plan; a pre-treatment planning computed tomography image parameter; a body region of the patient.

13. The radiation therapy device according to Claim 11 or 12, wherein the knowledge-based model includes at least one of an expert system and a physical model.

14. The radiation therapy device according to Claim 11, 12 or 13, wherein the plurality of imaging parameters comprises at least one of a source voltage and a source current of the plurality of radiation sources.

15. The radiation therapy device according to Claim 11, 12, 13 or 14, wherein the plurality of imaging parameters comprises an image capture parameter of the plurality of imagers.
